Europäisches Patentamt

European Patent Office

Office européen des brevets

(19)

(11) Veröffentlichungsnummer : **0 074 359**
**B1**

# (12) EUROPÄISCHE PATENTSCHRIFT

(45) Veröffentlichungstag der Patentschrift :
**09.01.85**

(51) Int. Cl.⁴ : **G 01 L  5/06**, G 01 N  3/20

(21) Anmeldenummer : **82900619.6**

(22) Anmeldetag : **04.03.82**

(86) Internationale Anmeldenummer :
**PCT/CH 82/00034**

(87) Internationale Veröffentlichungsnummer :
**WO/8203274 (30.09.82 Gazette 82/23)**

(54) **PRÜFGERÄT FÜR TENNISSCHLÄGER.**

(30) Priorität : **23.03.81 CH 1963/81**

(43) Veröffentlichungstag der Anmeldung :
**23.03.83 Patentblatt 83/12**

(45) Bekanntmachung des Hinweises auf die Patenterteilung : **09.01.85 Patentblatt 85/02**

(84) Benannte Vertragsstaaten :
**AT BE DE FR GB NL SE**

(56) Entgegenhaltungen :
**DE-A- 2 916 735**
**FR-A-  567 476**
**US-A- 2 299 722**

(73) Patentinhaber : **WEIDMANN, Ulrich**
**Industriestrasse 13**
**CH-8152 Glattbrugg (CH)**

(72) Erfinder : **WEBER, Hans Rudolf**
**Althoossteig 11**
**CH-8046 Zürich (CH)**
Erfinder : **WEIDMANN, Ulrich**
**Industriestrasse 13**
**CH-8152 Glattbrugg (CH)**

(74) Vertreter : **Feldmann, Clarence Paul et al**
**c/o Patentanwaltsbüro FELDMANN AG Postfach**
**Kanalstrasse 17**
**CH-8152 Glattbrugg (CH)**

## Beschreibung

Die Erfindung bezieht sich auf ein Prüfgerät für Tennischläger der im Oberbegriff des Anspruchs 1 genannten Art. Ein solches Gerät ist aus DE-A-2 916 735 bekannt.

Die Bespannung der Schlagfläche eines Tennisschlägers kann mehr oder weniger hart ausgeführt sein. Je höher die Spannung der Saiten, umso steifer ist die Schlagfläche. Die Saiten können aus einem einzigen verstreckten Kunststoffaden bestehen oder aus mehreren verdrillten Einzelfäden aufgebaut sein. Statt aus Kunststoffsaiten kann die Bespannung aus Darmsaiten bestehen. Die Kunststoffbespannung hat die grössere Verschleissfestigkeit.

Je nachdem reagiert eine Kunststoffbespannung bei scharfen Schlägen hart und bei schwachen Schlägen weich. Die Bespannung hat eine sehr wichtige Funktion. Je nach Führung des Schlägers schleudert sie den Ball mit unterschiedlicher Geschwindigkeit und Rotation zurück. Dabei verformen sich kurzzeitig, während einiger tausendstel Sekunden, Ball, Bespannung und Schläger. Vom Schläger verformt sich insbesondere der Rahmen. Alle diese Faktoren zusammen, Härte des Balles, Härte der Bespannung, Elastizität des Rahmens und Führung des Schlägers bestimmen die Ballkontrolle.

Ein Turnierspieler hat daher meist mehrere Schläger und kann beurteilen, welcher Schläger dem Spielverhalten des Gegeners, dem Ball und den Bodenverhältnissen am besten angepasst ist. Statt die Härte gefühlsmässig zu bestimmen, ist es für den geübten Spieler von Vorteil, wenn die Härte der Bespannung gemessen werden kann.

### Stand der Technik

Zum Messen der Bespannung sind verschiedene Geräte bekannt geworden. Bei einem Gerät wird die Messvorrichtung auf dem Rahmen selber befestigt·und die Härte der Bespannung mittels eines durch ein Gewicht belasteten Hebels gemessen. Eine solche Messung hat den Nachteil, dass in das Messresultat nicht nur die Härte der Bespannung sondern auch die Elastizität des Rahmens eingeht.

### Aufgabe der Erfindung

Die Erfindung stellt sich zur Aufgabe, ein Prüfgerät zu schaffen, welches den genannten Nachteil vermeidet, sodass die Messung die Härte der Bespannung anzeigt, ohne die Steifheit oder Elastizität des Rahmens mit einzubeziehen. Da die Steifheit des Rahmens eine wichtige Rolle spielt, soll diese für sich in bespanntem oder unbespanntem Zustand messbar sein. Schliesslich soll auch die Lage des Schwerpunktes mit Hilfe des Geräts feststellbar sein.

Das Gerät gemäss Oberbegriff des Anspruches 1, zeichnet sich erfindungsgemäß dadurch aus, dass die Auflageelemente aus zwei abnehmbaren, den Rahmen jeweils im seitlichen Bereich unterstützenden Auflageleisten bestehen, deren Abstand voneinander zur Anpassung an den Rahmen und Zentrierung desselben einstellbar ist, in dem dem Lagerbock gegenüberliegenden Bereich der Fundamentplatte ein den Griff des zu prüfenden Tennisschlägers in vertikaler Richtung fixierendes Widerlager vorgesehen ist, auf der Fundamentplatte im mittleren Bereich zwischen Lagerbock und Widerlager eine in ihrer Position verstellbare, sich quer zum Tennisschläger erstreckende Stützleiste lösbar befestigt ist, und daß die Anzeigevorrichtungen eine am Lagerbock befestigte Messuhr umfassen, deren Messstifte auf dem Hebel aufliegt.

Für die Messungen der Härte der Bespannung muss der Rahmen genau zentrisch unter dem Stempel liegen, wozu die Auflageleisten senkrecht zu ihrer Längsrichtung in Schlitzen der Fundamentplatte mittels eines Führungsmechanismus verstellbar sein können, der mittels durch die Schlitze hindurchragender und in die Auflageleisten eingreifender Stifte eine gegenläufige Bewegung der Auflageleisten steuert. Schliesslich kann die Messung des Schwerpunktes des Schlägers dadurch realisiert werden, dass das Widerlager eine Brücke umfasst, die zwischen zwei auf der Fundamentplatte befestigten Lagern um eine horizontale Achse schwenkbar gehalten ist und oben eine Auflageplatte trägt.

### Die Zeichnung

In der Zeichnung ist ein Ausführungsbeispiel des Erfindungsgegenstandes dargestellt. An Hand derselben ist anschliessend dessen Verwendung erläutert.

Die Zeichnungen zeigen :

Figur 1 ein Prüfgerät von der Seite beim Messen der Härte der Bespannung,

Figur 2 das Gerät nach Figur 1 in Ansicht von oben,

Figur 3 das Gerät nach Figur 1, in umgerüstetem Zustand zum Prüfen der Steifheit des Rahmens,

Figur 4 das Gerät nach Figur 1 in Ansicht von unten und

Figur 5 das Gerät nach Figur 1 beim Feststellen der Schwerpunktslage eines Tennisschlägers.

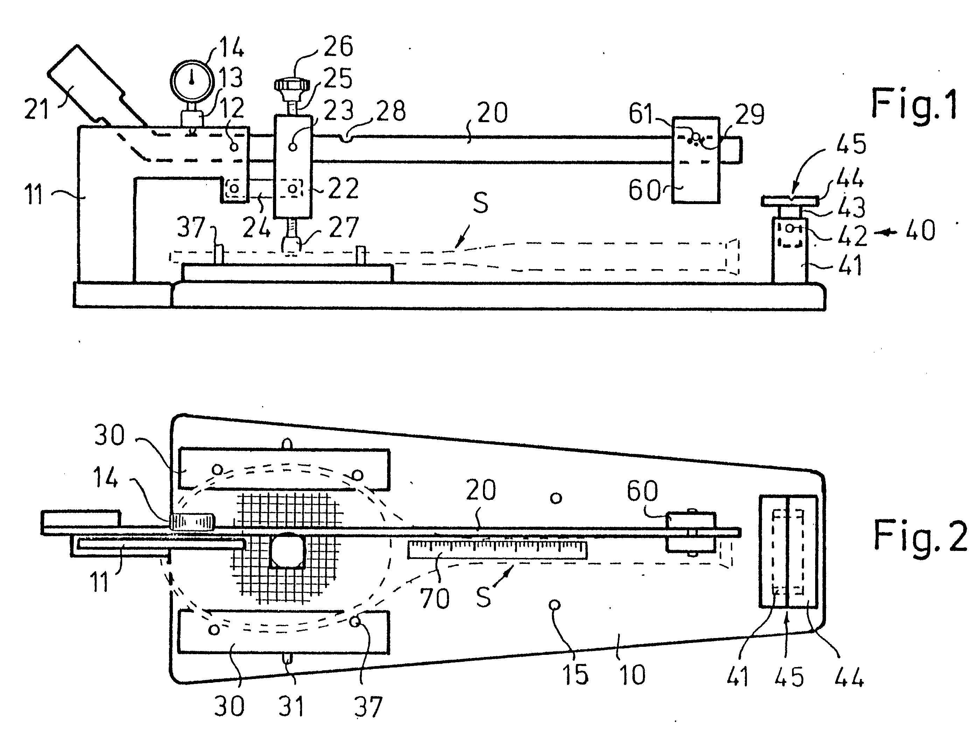

Figur 1 und 2 zeigen das Gerät in der Ausrüstung wie dies zum Messen der Bespannungshärte erforderlich ist. Es umfasst eine Fundamentplatte 10 in Form eines langgestreckten Trapezes. An der breiten Parallelseite ist ein Lagerbock 11 befestigt, an dem ein Hebel 20 bei 12 schwenkbar befestigt ist. Der Hebel ist an seinem kürzeren Ende mit einem Ausgleichsgewicht 21 versehen, das das Gewicht des Hebels in Horizontallage teilweise ausbalanciert. Am Hebel ist ein Block 22 bei 23 angelenkt. Er ist mittels einer am Block 22 und am Lagerbock 11 angelenkten Lasche 24 bezüglich Vertikalbewegungen parallel geführt. Im Block 22 ist eine Stellschraube 25 angebracht, die oben einen Knopf 26 und unten einem Stempel 27 trägt. Am Lagerbock 11 ist ein Halter 13 befestigt für eine Messuhr 14, deren Messtift auf dem Hebel 20 aufliegt.

Der Abstand des Drehpunktes 12 des Hebels 20 zum Anlenkpunkt 23 ist gleich gross wie der Abstand des Drehpunktes 12 zur Auflagestelle des Messtiftes, damit die Senkung des Stempels an der Messuhr ablesbar ist. An der oberen Seite des Hebels 20 sind zwei Kerben 28 und 29 angebracht. Auf der Fundamentplatte 10 sind zwei Auflageleisten 30 wegnehmbar angebracht.

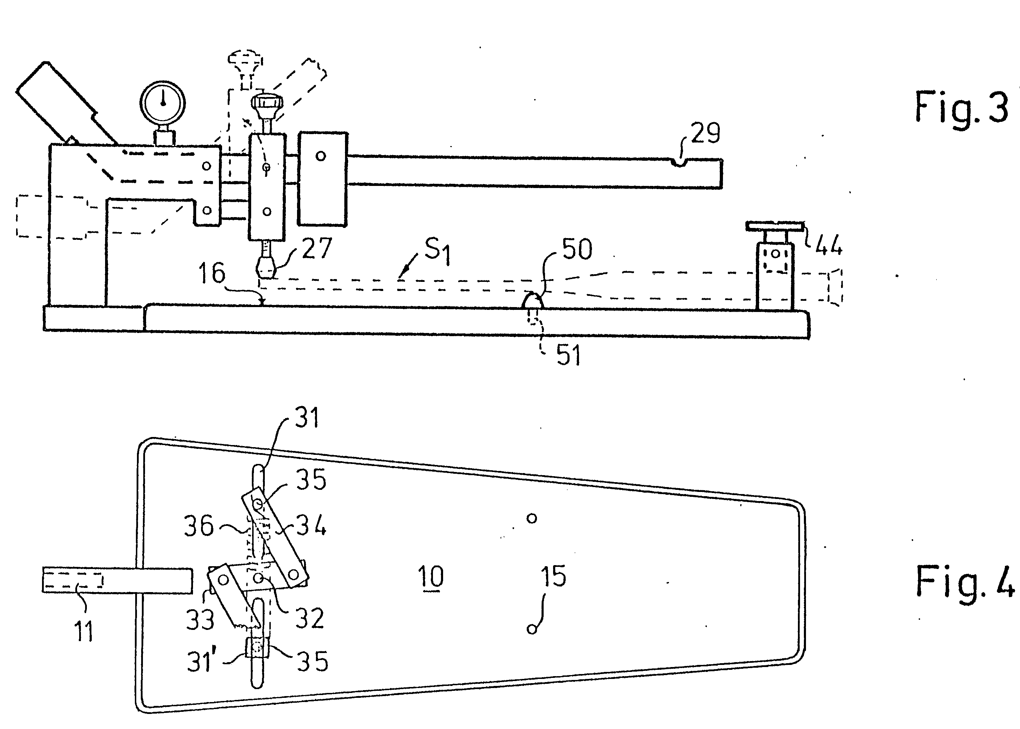

Sie sind dazu mit nach unten ragenden, nicht dargestellten Zapfen versehen, die in Schieber eingreifen. Die Schieber sind in den senkrecht zur Längsachse der Fundamentplatte verlaufenden Nuten 31 gleitend geführt. Damit sich die Auflageleisten 30 bei ihrer Verschiebung immer gleich weit von der Mittellinie der Fundamentplatte entfernen, sind die Schieber mittels einem, unter der Grundplatte angeordneten Führungsgestänge versehen, das in Figur 4 ersichtlich ist. Der Drehpunkt 32 ist fest mit der Fundamentplatte 10 verbunden. Er trägt einen zweiarmigen Hebel 33 mit gleich langen Armen. An ihm sind zwei Laschen 34 angelenkt und mit Zapfen 35 der in den Nuten 31 geführten Schieber 31' verbunden. Die unteren Enden der Zapfen 35 sind durch eine Zugfeder miteinander verbunden.

An ihrer oberen Seite sind die Auflageleisten je mit zwei nach oben ragenden Zentrierstiften 37 versehen, die den Rahmen eines auf die Auflageleisten gelegten Tennisschlägers zentrieren.

Im Bereich der schmalen Parallelseite der Fundementplatte 10 ist ein Widerlager 40 befestigt. Es umfasst eine in den Lagern 41 um eine horizontale Achse 42 schwenkbare Brücke 43. Die Brücke trägt oben eine Auflageplatte 44, die mit einer parallel zur Schwenkachse 42 verlaufenden Markierungslinie 45 versehen ist.

Zwischen dem Lagerbock 11 und dem Widerlager 40 sind in der Fundamentplatte 10 Bohrungen 15 angebracht, in die die Stifte 51 einer quer zur Längsrichtung der Fundamentplatte verlaufenden Stützleiste 50 einsetzbar sind.

Schliesslich ist mit 60 ein Gewicht bezeichnet, das eine tiefe Nute und einen quer dazu verlaufenden Stift 61 hat und 70 stellt einen auf die Fundmentplatte 10 angebrachten Masstab dar.

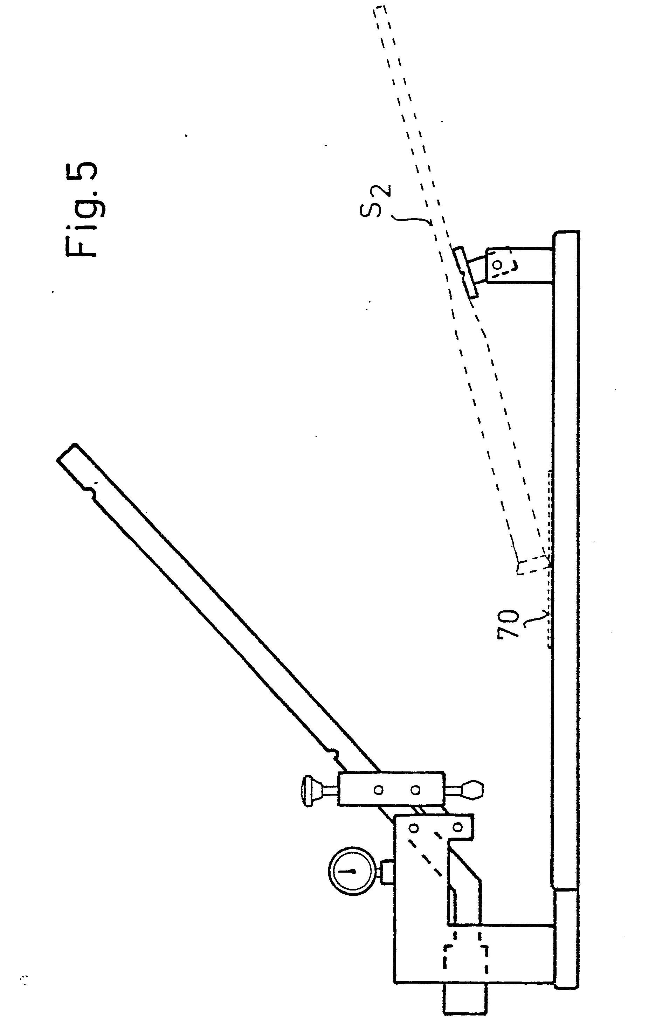

Bei Nichtgebrauch, im Ruhestand ist der Hebel um etwa 45° nach oben geschwenkt, wie dies in Figur 5 dargestellt ist. In dieser Lage ist er durch einen Riegel oder einen Magneten gehalten.

Verwendung des Geräts beim Messen der Härte der Bespannung (Figur 1 und 2)

Ein Tennisschläger S wird auf die beiden voneinander entfernten Auflageleisten 30 gelegt, wobei die Stifte 37 den Rahmen zentrieren, so dass die Mitte des Rahmens unter dem Stössel 27 liegt. Nun wird der Hebel 20 vorsichtig nach unten bewegt, bis der Stössel auf der Bespannung aufliegt. Das teilweise durch das Gegengewicht 21 kompensierte Gewicht des Hebels 20 erzeugt dabei eine Vorspannung von etwa 2 kg. Diese Vorspannung bewirkt, dass der Rahmen satt auf den Auflageleisten aufliegt, sodass genau vergleichbare und zuverlässige Messresultate erzielt werden. Durch Drehen der Stellschraube 25 mittels des Knopfes 26 lässt sich die Messuhr auf Null stellen. Nun wird das Belastungsgewicht 60 auf den Hebel 20 geschoben, bis Stift 61 in die Kerbe 29 einrastet. Die Bespannung ist jetzt voll belastet und die Senktiefe des Stössels ist an der Messuhr 14 ablesbar. Je härter die Bespannung ist, je weniger senkt sich der Stössel 27. Auf diese Weise kann die Bespannungshärte genau wiederholbar und registrierbar gemessen werden.

Verwendung des Geräts beim Messen der Steifheit des Rahmens (Figur 3)

Zum Messen der Steifheit des Rahmens muss das Gerät umgerüstet werden. Zuerst müssen die beiden Auflageleisten 30 weggenommen werden. Danach muss je nach der Grösse des Rahmens die Stützleiste 50 mit ihren Zentrierzapfen 51 in die vorderen oder rückseitigen Bohrungen 15 eingesetzt werden. Der Griff des Schlägers S 1 wird nun unter die Brücke 43 des Widerlagers 40 so weit hindurch geschoben, bis zu einer auf der Fundamentplatte angebrachten Markierungslinie 16. Dadurch liegt der obere Rand des Rahmens unter dem Stössel 27. Die Stützleiste 50 liegt jetzt im Bereich zwischen dem Rahmen und dem Griff. Jetzt kann man den Stössel herunter lassen bis er unter dem Gewicht des Hebels 20 wieder mit etwa zwei Kilo Vorspannung am Rand des Rahmens aufliegt.

Jetzt wird der Stössel 27 verstellt bis die Messuhr unter dieser Vorbelastung wieder auf Null steht. Da der Hebelarm, auf dem die Belastung ruht, jetzt viel grösser ist, darf die Belastung durch das Gewicht 60 nicht zu gross sein. Das Gewicht 60 wird daher bis zur Kerbe 28 aufgeschoben und dann die Senkung des Stössels auf der Messuhr abgelesen. Je steifer der Rahmen ist, desto geringer ist die Senkung des Stössels.

Verwendung des Gerätes zum Feststellen der Schwerpunktslage eines Schlägers (Figur 5)

Will man die Schwerpunktslage eines Schlägers S 2 feststellen, legt man ihn auf die Auflegeplatte 44 mit dem Griff zur Fundamentplatte hin gerichtet. Danach verschiebt man den Tennisschläger bis er sich gerade in der Kipplage befindet und der Griff von selber langsam auf die Fundamentplatte herab sinkt. Das Griffende ruht dann fast ohne Druck auf dem, auf der Fundamentplatte 10 angebrachten Masstab (siehe Figur 1 und Figur 5).

Auf diesem Masstab kann man dann direkt die Schwerpunktslage, das heisst deren Abstand vom Griffende ablesen.

## Ansprüche

1. Prüfgerät für Tennisschläger mit einer Fundamentplatte (10), einem am Rande der Fundamentplatte angeordneten Lagerbock (11), einem in dem Lagerbock (11) um eine horizontale Achse (12) drehbar gelagerten Hebel (20) mit einem sich über der Fundamentplatte (10) erstreckenden Hebelarm, einem an dem Hebelarm an dessen dem Drehpunkt zugewandten Ende angelenkten und bei Verschwenkung des Hebelarms in vertikaler Richtung bewegten Stempel (27), einem Gewicht (60) zur Belastung des Hebelarms, auf der Fundamentplatte (10) angeordneten einstellbaren Auflageelementen (30) zur Abstützung des Rahmens eines zu prüfenden Tennisschlägers (S ; S1) in horizontaler Lage, derart, dass mit dem Stempel die Mitte der Bespannung belastbar ist, sowie Anzeigeeinrichtungen (14) zur Ableitung einer die Härte der Bespannung repräsentierenden Anzeige anhand einer vom Stempel unter dem Einfluss des Gewichts verursachten Durchbiegung der Bespannung, dadurch gekennzeichnet, dass die Auflageelemente aus zwei abnehmbaren, den Rahmen jeweils im seitlichen Bereich unterstützenden Auflageleisten (30) bestehen, deren Abstand voneinander zur Anpassung an den Rahmen und Zentrierung desselben einstellbar ist, in dem dem Lagerbock (11) gegenüberliegenden Bereich der Fundamentplatte (10) ein den Griff des zu prüfenden Tennisschlägers (S ; S1) in vertikaler Richtung fixierendes Widerlager (40) vorgesehen ist, auf der Fundamentplatte (10) im mittleren Bereich zwischen Lagerbock (11) und Widerlager eine in ihrer Position verstellbare, sich quer zum Tennisschläger erstreckende Stützleiste (50) lösbar befestigt ist, und daß die Anzeigeeinrichtungen eine am Lagerbock befestigte Messuhr (14) umfassen, deren Messstift auf dem Hebel (20) aufliegt.

2. Prüfgerät nach Anspruch 1, dadurch gekennzeichnet, dass das Widerlager (40) eine Brücke (43) umfasst, die zwischen zwei auf der Fundamentplatte befestigten Lagern (41) um eine horizontale Achse (42) schwenkbar gehalten ist und oben eine Auflageplatte (44) trägt.

3. Prüfgerät nach Anspruch 1, dadurch gekennzeichnet, dass der Stempel (27) mittels einer Parallelführung (24) mit dem Lagerbock (11) verbunden ist.

4. Prüfgerät nach Anspruch 1, dadurch gekennzeichnet, dass sich die Anlenkstelle (23) des Stempels (27) am Hebelarm gleich weit vor dem Drehpunkt (12) des Hebels (20) befindet wie die Berührungsstelle des Messstiftes der Messuhr (14) sich hinter diesem Drehpunkt befindet.

5. Prüfgerät nach Anspruch 1, dadurch gekennzeichnet, dass die Auflageleisten (30) in Schlitzen (31) der Fundamentplatte (10) mittels eines Führungsmechanismus (32-36) verstellbar sind, der mittels durch die Schlitze (31) hindurchragender und in die Auflageleisten (30) eingreifender Stifte (35) eine gegenläufige Bewegung der Auflageleisten steuert.

## Claims

1. A testing apparatus for tennis rackets, having a base plate (10), a bearing block (11) disposed at the edge of the base plate, a lever (20) mounted in the bearing block (11) so as to be rotatable about a horizontal axis (12) and having a lever arm extending beyond the base plate (10), a piston (27) articulated on the lever arm at its end facing the point of rotation and moved in the vertical direction when the lever arm is pivoted, a weight (60) for loading the lever arm, adjustable bearing elements (30) disposed on the base plate (10) for supporting the frame of a tennis racket (S ; S1) to be tested in a horizontal position in such a way that the centre of the stringing may be stressed by the piston, and display devices (14) for producing a display representing the hardness of the stringing by way of a bending of the stringing caused by the piston under the influence of the weight, characterized in that the bearing elements comprise two detachable bearing strips (30) which each support the frame in the lateral region and the distance between which may be adjusted for fitting to the frame and centring the latter, a support (40) fixing the handle of the tennis racket (S ; S1) to be tested in the vertical direction is provided in the area of the base plate (10) opposite the bearing block (11), a supporting strip (50) which extends transversely to the tennis racket and the position of which may be adjusted is detachably secured to the base plate (10) in the central region between the bearing block (11) and the support, and the display devices comprise a measuring gauge (14) which is secured to the bearing block and the measuring pin of which bears against the lever (20).

2. A testing apparatus according to Claim 1, characterized in that the support (40) comprises a bridge (43) which is held pivotably about a horizontal axis (42) between two bearings (41) secured to the base plate and at its top supports a bearing plate (44).

3. A testing apparatus according to Claim 1, characterized in that the piston (27) is connected to the bearing block (11) by means of a parallel guide (24).

4. A testing apparatus according to Claim 1, characterized in that the pivoting point (23) of the piston (27) on the lever arm is as far in front of the point of rotation (12) of the lever (20) as the point of contact of the measuring pin of the measuring gauge (14) is behind the said point of rotation.

5. A testing apparatus according to Claim 1, characterized in that the bearing strips (30) are displaceable in slots (31) in the base plate (10) by means of a guide mechanism (32 to 36) which controls an opposite movement of the bearing strips by means of pins (35) projecting through the slots (31) and engaging in the bearing strips (30).

## Revendications

1. Appareil de contrôle pour raquette de tennis avec une plaque de base (10), un support d'appui (11) disposé au bord de la plaque de base, un levier (20) monté pivotant autour d'un axe horizontal (12) sur le support d'appui (11) et comportant un bras de levier s'étendant au-dessus de la plaque de base (10), un poussoir (27) articulé sur ce levier à son extrémité voisine de son point de rotation et se déplaçant en direction verticale lors du pivotement du bras de levier, un poids (60) chargeant ce bras de levier, des éléments d'appui réglables (30) disposés sur la plaque de base (10) pour soutenir en position horizontale le cadre d'une raquette de tennis (S, S1) à contrôler, de sorte que le milieu de la garniture en boyaux de la raquette puisse être chargé avec le poussoir, ainsi que des dispositifs indicateurs (14) pour obtenir une indication représentative de la dureté de la garniture à l'aide d'une flexion de cette garniture provoquée par le poussoir sous l'action du poids, appareil caractérisé en ce que les éléments d'appui sont constitués de deux barrettes d'appui amovibles (30) soutenant respectivement le cadre dans ses zones latérales, et dont la distance de l'une à l'autre est susceptible d'être réglée pour l'adapter au cadre et permettre le centrage de celui-ci, tandis qu'il est prévu dans la partie de la plaque de base (10) opposée au support d'appui (11) un contre-appui (40) bloquant en direction verticale la poignée de la raquette de tennis (S, S1) à contrôler, une barrette de support (50) s'étendant transversalement par rapport à la raquette de tennis et susceptible d'être réglée en position, étant fixée de façon amovible dans la zone médiane entre le support d'appui (11) et le contre-appui (40), et les dispositifs indicateurs comportant un comparateur (14) fixé sur le support d'appui et dont la broche de mesure s'applique sur le levier (20).

2. Appareil de contrôle selon la revendication 1, caractérisé en ce que le contre-appui (40) comporte un pont (43) maintenu de façon à pouvoir pivoter autour d'un axe horizontal (42) entre deux paliers (41) fixés sur la plaque de base, et qui porte sur le dessus une plaque d'appui (44).

3. Appareil de contrôle selon la revendication 1, caractérisé en ce que le poussoir (27) est relié au support d'appui (11) au moyen d'un guidage parallèle (24).

4. Appareil de contrôle selon la revendication 1, caractérisé en ce que le point d'articulation (23) du poussoir (27) sur le bras de levier, se situe en avant du point de rotation (12) du levier (20) à une distance égale à celle où se situe, en arrière de ce point de rotation, le point de contact de la broche de mesure du comparateur (14).

5. Appareil de contrôle selon la revendication 1, caractérisé en ce que les barrettes d'appui (30) sont susceptibles d'être réglées dans des fentes (31) de la plaque de base (10) au moyen d'un mécanisme de guidage (32 à 36) qui commande un déplacement réciproque des barrettes d'appui au moyen de broches (35) venant en prise dans les barrettes d'appui (30) en traversant les fentes (31).

Fig.1

Fig.2

0 074 359

Fig.3

Fig.4

0 074 359

Fig.5

S₂

70